# EUROPÄISCHE PATENTANMELDUNG

(11) **EP 3 991 677 A1**
(43) Veröffentlichungstag der Anmeldung: **04.05.2022**
(21) Anmeldenummer: 21199437.1
(22) Anmeldetag: 28.09.2021
(51) Int. Cl.: A61B 18/12, A61B 18/14

(54) **ELEKTROCHIRURGIESYSTEM MIT ABSAUGEINRICHTUNG**

(30) Priorität: 30.10.2020 DE 102020128693
(71) Anmelder: Olympus Winter & Ibe GmbH, 22045 Hamburg (DE)
(72) Erfinder: Janich, Fabian, 14469 Potsdam (DE); Breitsprecher, Frank, 12527 Berlin (DE); Krüger, Jens, 15732 Eichwalde (DE)
(74) Vertreter: Eisenführ Speiser

(57) **Zusammenfassung**

Die Erfindung betrifft ein Elektrochirurgiesystem (10) mit einem elektrochirurgischen Instrument (14), einer Absaugeinrichtung (26) und einem Elektrochirurgie-Generator (12), an den das elektrochirurgische Instrument (14) und die Absaugeinrichtung (26) angeschlossen sind, wobei der Elektrochirurgie-Generator (12) einen Prozessor (70) und mindestens einen Speicher (72) aufweist und dazu konfiguriert ist, Einträge in dem Speicher (74, 76, 78) in Steueranweisungen für den Betrieb der Absaugeinrichtung (26) umzusetzen und im Betrieb den Steueranweisungen entsprechende Steuereingriffe zur Ansteuerung der Absaugeinrichtung (26) vorzunehmen, wobei die Steueranweisungen für den Betrieb der Absaugeinrichtung (26) definierenden Einträge in dem Speicher (74, 76, 78) für ein jeweiliges elektrochirurgische Instrument (14) und/oder einen jeweiligen Betriebsmodus spezifisch sind.

## Beschreibung

Die Erfindung betrifft ein Elektrochirurgiesystem mit einem Elektrochirurgie-Generator, der dazu konfiguriert ist, ein elektrochirurgisches Instrument mit hochfrequentem Wechselstrom zu versorgen und eine Absaugeinrichtung anzusteuern. Die Erfindung betrifft außerdem ein elektrochirurgisches Instrument und einen Elektrochirurgie-Generator.

Ein Elektrochirurgiesystem umfasst in der Regel einen Elektrochirurgie-Generator zum Erzeugen des hochfrequenten Wechselstroms. Der Elektrochirurgie-Generator hat in der Regel zwei oder mehr Ausgänge, an die ein elektrochirurgisches Instrument angeschlossen werden kann und zwischen denen im Betrieb eine hochfrequente Wechselspannung bereitgestellt wird.

An den Elektrochirurgie-Generator können typischerweise verschiedene elektrochirurgische Instrumente für unterschiedliche Aufgaben sowie eine Absaugeinrichtung, insbesondere eine Rauchgasabsaugeinrichtung angeschlossen werden.

Mittels Elektrochirurgie kann biologisches Gewebe - also Körpergewebe - geschnitten, koaguliert (verödet) und/oder vaporisiert werden. Für die Elektrochirurgie werden typischerweise hochfrequente Wechselströme mit einer Frequenz zwischen 0,2 MHz und 3 MHz verwendet. Elektrochirurgische Instrumente sind typischerweise Handinstrumente, mit denen ein Arzt Körpergewebe koagulieren, ablatieren und/oder schneiden kann.

Elektrochirurgische Instrumente werden hierzu mit hochfrequenter elektrischer Energie gespeist, mit der Gewebe gezielt koaguliert oder geschnitten werden kann. Die hochfrequente elektrische Energie wird von dem Elektrochirurgie-Generator bereitgestellt und mittels des jeweils geeigneten elektrochirurgischen Instruments auf Körpergewebe angewandt. Je nach Anwendung sind spezielle Strom- und Spannungsverläufe notwendig, die dem Arzt in Form von Betriebsmodi (auch Modes genannt) zur Auswahl am Elektrochirurgie-Generator zur Verfügung gestellt werden. Diese Betriebsmodi können fest im Elektrochirurgie-Generator hinterlegt sein.

Während der Anwendung eines elektrochirurgischen Instruments können je nach Instrument und Betriebsmodus beispielsweise Rauchgase entstehen. Da diese störend oder unter Umständen auch schädlich sein können, können bekannte Elektrochirurgie-Generatoren in Verbindung mit einer Absaugeinrichtung, insbesondere mit einer Rauchgasabsaugeinrichtung verwendet werden. Das An- und Abschalten der Rauchgasabsaugeinrichtung kann durch den Elektrochirurgie-Generator gesteuert erfolgen.

Der Erfindung liegt die Aufgabe zugrunde, ein Elektrochirurgiesystem zu schaffen, das eine verbesserte Ansteuerung einer Absaugeinrichtung bietet.

Erfindungsgemäß werden hierzu ein Elektrochirurgiesystem, ein Elektrochirurgie-Generator und ein elektrochirurgisches Instrument vorgeschlagen, die jeweils für sich und in Kombination miteinander verschiedene auf einen jeweiligen Anwendungsfall bzw. für ein jeweiliges elektrochirurgisches Instrument maßgeschneiderte Betriebsmodi und eine jeweils passende Ansteuerung einer Absaugeinrichtung ermöglichen.

Ein erfindungsgemäßes Elektrochirurgiesystem umfasst ein elektrochirurgisches Instrument und einen Elektrochirurgie-Generator, an den das elektrochirurgische Instrument sowie eine Absaugeinrichtung im Betrieb angeschlossen sind.

Der Elektrochirurgie-Generator verfügt über einen Prozessor und mindestens einem Generator-Datenspeicher, in dem ein Betriebsprogramm zur Steuerung des Betriebs des Elektrochirurgie-Generators in Verbindung mit dem elektrochirurgischen Instrument und der Absaugeinrichtung gespeichert ist.

Der Prozessor ist mittels des Betriebsprogramms dazu konfiguriert, für unterschiedliche elektrochirurgische Instrumente und/oder unterschiedliche Betriebsmodi jeweils spezifische Steueranweisungen einzulesen und deren Umsetzung in Steuereingriffe an dem Elektrochirurgie-Generator, an dem elektrochirurgischen Instrument und/oder an der Absaugeinrichtung zu bewirken. Steuereingriffe umfassen dabei beispielsweise das Steuern der Abgabe von Energie an ein im Betrieb an den Elektrochirurgie-Generator angeschlossenes elektrochirurgisches Instrument. Steuereingriffe können aber auch das Ein- oder Ausschalten der Absaugeinrichtung sein, so dass beispielsweise über ein schnell aufeinanderfolgendes Ein- und Ausschalten der Absaugeirichtung eine Leistungssteuerung im Sinne einer Pulsweitenmodulation möglich ist. Im Betrieb setzt der Elektrochirurgie-Generator somit Steueranweisungen in Steuereingriffe um. Hierzu wird der Elektrochirurgie-Generator durch den Prozessor und das in dem Generator-Datenspeicher gespeicherte Betriebsprogramm gesteuert.

Erfindungsgemäß wird somit ein Elektrochirurgiesystem mit einem elektrochirurgischen Instrument, einer Absaugeinrichtung und einem Elektrochirurgie-Generator vorgeschlagen, an den das elektrochirurgische Instrument und die Absaugeinrichtung angeschlossen sind, wobei der Elektrochirurgie-Generator einen Prozessor und mindestens einen Datenspeicher aufweist und dazu konfiguriert ist, Einträge in dem Datenspeicher in Steueranweisungen für den Betrieb der Absaugeinrichtung umzusetzen und im Betrieb den Steueranweisungen entsprechende Steuereingriffe zur Ansteuerung der Absaugeinrichtung vorzunehmen, wobei die Einträge, die die Steueranweisungen für den Betrieb der Absaugeinrichtung definieren, für ein jeweiliges elektrochirurgische Instrument und/oder einen jeweiligen Betriebsmodus spezifisch sind.

Die Im Betrieb von dem Prozessor durch das Betriebsprogramm gesteuert einzulesenden, für ein jeweiliges elektrochirurgisches Instrument und/oder einen jeweiligen Betriebsmodus spezifischen Steueranweisungen können direkt oder indirekt durch in einem Generator-Datenspeicher des Elektrochirurgie-Generators und/oder einem Instrumenten-Datenspeicher des elektrochirurgischen Instruments gespeicherte Einträge definiert sein. Erfindungsgemäß sind Einträge, die Steueranweisungen für Steuereingriffe an der Absaugeinrichtung definieren, jeweils einem Betriebsmodus und/oder einem elektrochirurgischen Instrument zugeordnet gespeichert. Die durch gespeicherte Einträge definierten Steueranweisungen für Steuereingriffe an der Absaugeinrichtung können bedingt sein oder von jeweils aktuellen Betriebszuständen des Elektrochirurgie-Generators oder von aktuell auftretenden Betriebsparameterwerten (wie z.B. der effektiv von dem elektrochirurgischen Instrument abgegeben Leistung) dynamisch abhängige Steuereingriffe definieren.

Der Generator-Datenspeicher des Elektrochirurgie-Generators enthält vorzugsweise eine theoretische beliebige Anzahl von Betriebsvorgaben, die von dem Betriebsprogramm aufgerufen werden können und den Betrieb des Elektrochirurgie-Generator beeinflussen können, aber keine festen Betriebsabläufe vorgeben. Die Betriebsvorgaben können einerseits Steuerbefehle, andererseits aber auch parametrische Daten sein, wie zum Beispiel Werte für die Ausgangsspannung, den Ausgangsstrom, die Absaugleistung der Absaugeinrichtung, Zeiteinstellungen, Schwellwerte, Fehlerbedingungen usw.

Die Betriebsvorgaben in dem Generator-Datenspeicher können als Bibliothek einer beliebigen Vielzahl von möglichen Steuerbefehlen oder Parameterwerten verstanden werden, auf die das Betriebsprogramm zugreifen kann. Allerdings erfolgt der Zugriff gemäß der bevorzugten Ausführungsvariante nicht unmittelbar, so dass die Betriebsweise des Elektrochirurgie-Generators allein durch die Betriebsvorgaben und das Betriebsprogramm in dem Generator-Datenspeicher noch nicht bestimmt ist.

Vielmehr ist vorzugsweise zusätzlich eine Datenstruktur vorgesehen, in der beliebige Datensätze mit strukturierten Daten gespeichert sind. Die strukturierten Daten enthalten Verweise auf konkrete Betriebsvorgaben in dem Generator-Datenspeicher, also auf konkrete Steuerbefehle und/oder Parameterwerte. Die strukturierten Daten enthalten die vorgenannten Einträge, die die Steueranweisungen definieren, welche von dem Prozessor auf Basis des Betriebsprogramms in Steuereingriffe an dem Elektrochirurgie-Generator oder der Absaugeinrichtung umgesetzt werden. Diese die Steueranweisungen definierenden Einträge sind somit in der Datenstruktur gespeichert.

Die beispielsweise durch Einträge in den strukturierten Daten definierten Steueranweisungen, die von dem Elektrochirurgie-Generator durch den Prozessor in Verbindung mit dem Betriebsprogramm gesteuert in Steuereingriffe umgesetzt werden, sind durch Steuerbefehle und gegebenenfalls zugeordnete Parameterwerte definiert. Die in den strukturierten Daten enthaltenen Verweise auf Betriebsvorgaben werden im Betrieb des Elektrochirurgie-Generators von dem dann ablaufenden Betriebsprogramm aufgerufen und infolgedessen diejenigen Betriebsvorgaben angewandt, auf die die Verweise verweisen. Der durch das Betriebsprogramm gesteuerte Prozessor wendet die Betriebsvorgaben an und bewirkt entsprechende Steuereingriffe durch den Elektrochirurgie-Generator und oder der Absaugeinrichtung. Die Steuereingriffe können somit sowohl das jeweilige elektrochirurgische Instrument als auch die Absaugeinrichtung betreffen.

Die konkrete Betriebsweise des Elektrochirurgie-Generators in der bevorzugten Ausführungsform hängt somit von drei verschiedenen Arten von gespeicherten Daten ab, nämlich
- von den das Betriebsprogramm definierenden Daten in dem Generator-Datenspeicher,
- von den potentiell anzuwendenden Betriebsvorgaben in dem Generator-Datenspeicher, die beispielsweise Steuerbefehle oder Parameterwerte definieren, und von denen einige aufgrund von in der Datenstruktur enthaltenen Verweise konkret zur Anwendung kommen
- von den in der Datenstruktur gespeicherten Datensätzen mit strukturierten Daten, die die Verweise auf konkret anzuwendende Betriebsvorgaben enthalten und die während des Ablaufs des Betriebsprogramms von dem Prozessor aufgerufen und in konkret anzuwendende Betriebsvorgaben übersetzt werden.

Die in der Datenstruktur gespeicherten strukturierten Daten enthalten vorzugsweise Einträge, die entweder direkt oder mittels der Verweise und in der Datenstruktur und der Betriebsvorgaben in dem Generator-Datenspeicher Steueranweisungen definieren, die im Betrieb des Elektrochirurgie-Generators in Steuereingriffe an der Absaugeinrichtung umgesetzt werden. Die Steueranweisungen, die die Ansaugeinrichtung betreffen, können beispielsweise ein Ein- oder Ausschalten der Absaugeinrichtung oder für den eingeschalteten Zustand der Absaugeinrichtung die jeweils durch die Absaugeinrichtung abzugebende Leistung definieren.

Der Elektrochirurgie-Generator kann ausgebildet sein, solche Steueranweisungen, die die von der Absaugeinrichtung abzugebende Leistung definieren, in Steuereingriffe in Form von Impulsfolgen umzusetzen, die im Sinne einer Pulsweitenmodulation ein schnell aufeinanderfolgendes Ein- und Ausschalten der Absaugeirichtung bewirken, so dass diese im Ergebnis mit einer größeren oder kleineren Leistung betrieben wird. Eine im Rahmen der Pulsweitenmodulation abgegebene Folge schnell aufeinander folgender Einschalt-Impulse ist durch die Länge der Einschaltimpulse und die Länge der Pausen (Ausschaltzeiten) zwischen den einzelnen Einschaltimpulsen definiert. Je kürzer die Pausen (Ausschaltzeiten) und je länger die Einschaltimpulse sind, desto höher ist die im Ergebnis von der Absaugeinrichtung abgegebene Leistung.

In einer bevorzugten Ausführungsvariante kann der Elektrochirurgie-Generator dazu ausgebildet sein, die von der Absaugeinrichtung im Betrieb abgegebene Leistung in Abhängigkeit eines berechneten oder eines sensorisch erfassten Parameterwertes zu regeln. Auch kann der Elektrochirurgie-Generator über Leistungserfassungsmittel zum Erfassen einer über ein elektrochirurgisches Instrument abgegebenen Leistung aufweisen, um die Absaugeinrichtung entsprechend der vom elektrochirurgischen Instrument abgegebenen Leistung steuern zu können.

Vorzugsweise ergeben sich die Steuereingriffe im Betrieb des Elektrochirurgie-Generators aus dem Betriebsprogramm in Verbindung mit den Betriebsvorgaben in dem Generator-Datenspeicher und den Verweisen oder Steueranweisungen in der Datenstruktur, die entweder in dem Instrumenten-Datenspeicher oder in dem Generator-Datenspeicher gespeichert ist. Das Betriebsprogramm, die Betriebsvorgaben und die Datenstruktur mit den Verweisen und Steueranweisungen können unabhängig voneinander geändert und vorgegeben werden, mit der Einschränkung, dass die Datenformate kompatibel sind. Die Betriebsweise des Elektrochirurgie-Generators in einem jeweiligen Betriebsmodus kann somit durch Ändern des in dem Generator-Datenspeicher gespeicherten Betriebsprogramms geändert werden, oder durch Ändern der Betriebsvorgaben oder durch Ändern der strukturierten Daten in der Datenstruktur oder auch durch eine Kombination dieser Änderungen.

Das elektrochirurgische Instrument enthält einen vorzugsweise nichtflüchtigen Instrumenten-Datenspeicher (z.B. ein EEPROM oder ähnliches), aber keinen Prozessor. Auf dem Instrumenten-Datenspeicher des chirurgischen Instruments befindet sich ein Datensatz mit strukturierten Daten. Die strukturierten Daten dieses Datensatzes sind zu der Datenstruktur in dem Generator-Datenspeicher des Elektrochirurgie-Generators kompatibel und können die Datenstruktur des Elektrochirurgie-Generators oder einen Teil der Datenstruktur des Elektrochirurgie-Generators bilden. Vorzugsweise enthält die Datenstruktur, die in dem nichtflüchtigen Instrumenten-Datenspeicher des elektrochirurgischen Instruments gespeichert ist, Einträge, die Steueranweisungen für Steuereingriffe an der Absaugeinrichtung definieren. Somit kann im Betrieb eine zu einem jeweiligen elektrochirurgischen Instrument und ggf. dem jeweiligen Betriebsmodus passende Ansteuerung der Absaugeinrichtung definiert werden.

Die Steueranweisungen für Steuereingriffe an der Absaugeinrichtung definierende Einträge enthaltende Datenstruktur kann somit Teil eines Generator-Datenspeichers sein, der physischer Bestandteil des Elektrochirurgie-Generators ist, oder sie wird durch den Inhalt des Instrumenten-Datenspeichers des elektrochirurgischen Instruments gebildet oder von einer Kombination von Inhalten des Generator-Datenspeichers des Elektrochirurgie-Generators und des Instrumenten-Datenspeichers des elektrochirurgischen Instruments.

Der Prozessor des Elektrochirurgie-Generators ist durch das Betriebsprogramm gesteuert dazu konfiguriert, den Instrumenten-Datenspeicher des elektrochirurgischen Instruments auszulesen, um auf diese Weise beispielsweise im Betrieb eine zum jeweiligen elektrochirurgischen Instrument passende Ansteuerung der Absaugeinrichtung sicherzustellen.

Der Prozessor des Elektrochirurgie-Generators kann in Verbindung mit dem Betriebsprogramm dazu konfiguriert sein, den Instrumenten-Datenspeicher des elektrochirurgischen Instruments auszulesen, nachdem das elektrochirurgische Instrument an den Elektrochirurgie-Generator angeschlossen wurde und bevor der Elektrochirurgie-Generator in einem Betriebsmodus betrieben wird. Der Prozessor des Elektrochirurgie-Generators kann in Verbindung mit dem Betriebsprogramm dazu konfiguriert sein, den Datensatz oder die Datensätze oder die in dem Datensatz oder den Datensätzen enthaltenen strukturierten Daten in eine Datenstruktur zu übertragen, die Teil des Elektrochirurgie-Generators ist.

Der Prozessor des Elektrochirurgie-Generators kann in Verbindung mit dem Betriebsprogramm alternativ dazu konfiguriert sein, den Instrumenten-Datenspeicher des elektrochirurgischen Instruments während des Ablaufs des Betriebsprogramms - also während der Elektrochirurgie-Generator in einem Betriebsmodus betrieben wird - auszulesen. In dem Instrumenten-Datenspeicher des chirurgischen Instruments ist somit kein Computerprogramm oder Algorithmus gespeichert, und die strukturierten Daten sind weder als Computerprogramm noch als ein mit einem Programm verbundener Algorithmus lesbar.

Die Struktur der strukturierten Daten erlaubt es den Verweisen Zeilennummern zuzuordnen. Die Zeilennummern erlauben den gezielten Aufruf der diesen zugeordneten Verweise mittels des Betriebsprogramms. Die Verweise wiederum verweisen eindeutig auf jeweils konkrete Betriebsvorgaben in dem Generator-Datenspeicher oder alternativ auch auf andere Verweise oder Steuerbefehle in den strukturierten Daten.

Anstelle explizit im Datensatz gespeicherte Zeilennummern vorzusehen, kann auch vorgesehen sein, dass die Zeilennummern erst beim Auslesen eines Datensatzes erzeugt werden, und zwar anhand der Struktur der die Verweise repräsentierenden strukturierten Daten in einem Datensatz. Dies ist dann möglich, wenn die Verweise beispielsweise in einer vorgesehenen Reihenfolge in einem Datensatz gespeichert sind.

Die strukturierten Daten liegen vorzugsweise in einem speichereffizienten Binärformat vor.

Vorzugsweise weist das Elektrochirurgiesystem eine Programmierschnittstelle oder mehrere Programmierschnittstellen auf, mittels der die Inhalte der Datenstruktur oder des Generator-Datenspeichers programmiert werden können. Entsprechend kann auch vorgesehen sein, dass das Betriebsprogramm in dem Generator-Datenspeicher über eine Programmierschnittstelle geändert werden kann, beispielsweise auf dem Wege eines Softwareupdates des Elektrochirurgie-Generators via USB.

Erfindungsgemäß wird auch ein Verfahren zum Betreiben eines Elektrochirurgie-Generators vorgeschlagen, demgemäß Betriebsvorgaben und eine Datenstruktur in voneinander unabhängig bereitgestellt sind, wobei die Datenstruktur Verweise auf die Betriebsvorgaben enthält und ein durch ein Betriebsprogramm gesteuerter Prozessor während des Ablaufs des Betriebsprogramms indirekt auf einzelne Betriebsvorgaben zugreift, indem zunächst ein Zugriff auf Verweise in der Datenstruktur erfolgt und daraufhin ein Abruf derjenigen Betriebsvorgabe oder Betriebsvorgaben erfolgt, auf die ein jeweiliger Verweis verweist.

Vorzugsweise wird die Datenstruktur aus einem Instrumenten-Datenspeicher eines elektrochirurgischen Instruments ausgelesen, und zwar vorzugsweise nach Anschluss und vor einer Anwendung eines elektrochirurgischen Instruments. Die in der Datenstruktur auf dem elektrochirurgischen Instrument enthaltenen Datensätze mit strukturierten Daten können in eine Datenstruktur übertragen werden, die auf einem Elektrochirurgie-Generator gespeichert ist.

Ein weiterer Aspekt der Erfindung ist ein Elektrochirurgie-Generator für ein Elektrochirurgiesystem der genannten Art. Der Elektrochirurgie-Generator besitzt Anschlüsse zum Anschließen eines elektrochirurgischen Instruments sowie einen Prozessor und mindestens einen Generator-Datenspeicher, in dem
- erste Daten gespeichert sind, die ein Betriebsprogramm zur Steuerung des Betriebs des Elektrochirurgie-Generators in Verbindung mit dem elektrochirurgischen Instrument definieren,
- zweite Daten gespeichert sind, die Betriebsvorgaben definieren, die von dem Betriebsprogramm aufgerufen werden können und den Betrieb des Elektrochirurgie-Generator beeinflussen können, aber keine festen Betriebsabläufe vorgeben, und
- dritte Daten, die eine Datenstruktur definieren, die Datensätze mit strukturierten Daten enthält, die Verweise auf in dem Generator-Datenspeicher gespeicherte Betriebsvorgaben enthalten.

Ein derartiger Elektrochirurgie-Generator kann leicht mit verschiedenen unterschiedlichen elektrochirurgischen Instrumenten verwendet werden, wobei die Anpassung ein jeweiliges elektrochirurgisches Instrument allein durch entsprechende, Verweise oder Steueranweisungen repräsentierende Einträge in der Datenstruktur erfolgen kann, ohne dass die Betriebsvorgaben in dem Generator-Datenspeicher des Elektrochirurgie-Generators geändert werden müssen.

Vorzugsweise enthält die Datenstruktur neben den in ihr gespeicherten Verweisen auch diesen Verweisen zugeordnete Zeilennummern, die im Betrieb ein gezieltes Aufrufen einzelner Verweise durch das Betriebsprogramm erlauben.

Ein weiterer Aspekt der Erfindung ist ein elektrochirurgisches Instrument, das einen Instrumenten-Datenspeicher aufweist, der Datensätze mit strukturierten Daten enthält, die Verweise auf in dem Generator-Datenspeicher gespeicherte Betriebsvorgaben enthalten. Ein derartiges elektrochirurgisches Instrument kann somit die nötigen Informationen enthalten, die eine auf das jeweilige elektrochirurgische Instrument abgestimmten Betriebsmodus erlauben, ohne dass dieser vollständig durch das elektrochirurgische Instrument definiert zu sein braucht.

Die Erfindung soll nun anhand von Ausführungsbeispielen mit Bezug auf die Figuren näher erläutert werden. Von den Figuren zeigt:
- Figur 1:: ein Elektrochirurgiesystem mit einem Elektrochirurgie-Generator und einem an diesem angeschlossenen elektrochirurgischen Instrument;
- Figur 2:: ein elektrochirurgisches Instrument;
- Figur 3:: ein schematisches Schaltbild eines Elektrochirurgie-Generators;
- Figur 4:: ein schematisches Bild eines Prozessors in Verbindung mit einem Generator-Datenspeicher des Elektrochirurgie-Generators aus Figur 3; und
- Figur 5:: ein schematisches Bild einer alternativen Konfiguration des Prozessors in Verbindung mit einem Generator-Datenspeicher des Elektrochirurgie-Generators aus Figur 3.

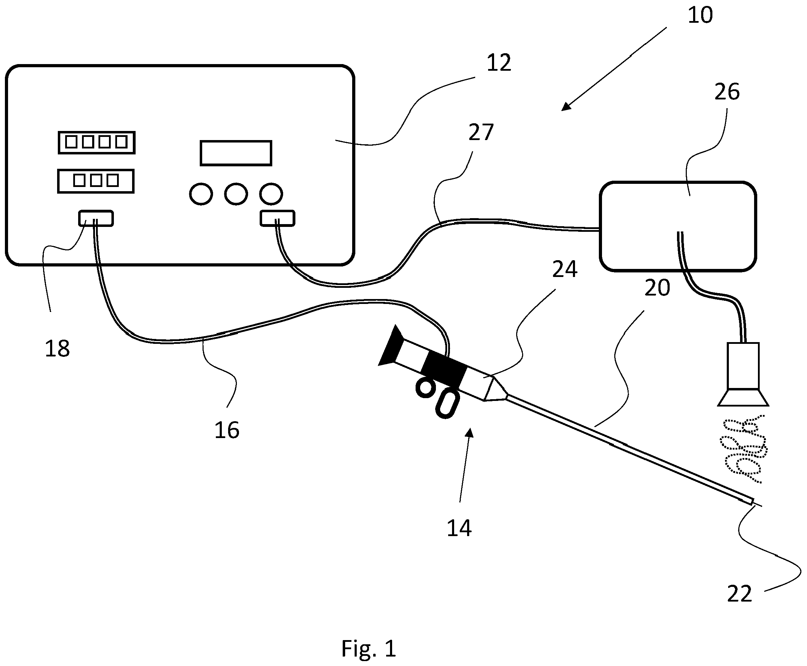

In Figur 1 ist ein Elektrochirurgiesystem 10 abgebildet. Das Elektrochirurgiesystem 10 umfasst einen Elektrochirurgie-Generator 12 und ein elektrochirurgisches Instrument 14. Das elektrochirurgisches Instrument 14 ist über ein Anschlusskabel 16 mit elektrischen Aus- und Eingängen 18 des Elektrochirurgie-Generators 12 verbunden.

Das elektrochirurgische Instrument 14 weist einen Schaft 20 auf, an dessen Ende sich eine aktive Elektrode 22 befindet. Der Schaft 20 ist an einem Griff 24 des elektrochirurgischen Instruments 14 befestigt.

Außerdem ist eine Absaugeinrichtung 26 zur Rauchgasabsaugung an den Elektrochirurgie-Generator 12 angeschlossen. Über eine Steuerleitung 27 kann die Absaugeinrichtung 26 durch den Elektrochirurgie-Generator 12 gesteuert ein- und ausgeschaltet werden.

Das elektrochirurgische Instrument 14 weist einen Instrumenten-Datenspeicher 28 als nichtflüchtigen Datenspeicher für Daten auf. Der Instrumenten-Datenspeicher 28 ist nichtflüchtig und kann beispielsweise ein ROM (Read Only Memory) sein, beispielsweise ein EPROM *(electrically programmable read-only memory),* insbesondere ein EEPROM (electrically erasable programmable read-only memory). Ein EEPROM ist ein nichtflüchtiger Datenspeicher, der gelesen, beschrieben und schreibgeschützt werden kann. Der Instrumenten-Datenspeicher 28 ist beispielsweise in dem Handgriff 24 des elektrochirurgischen Instruments 14 untergebracht.

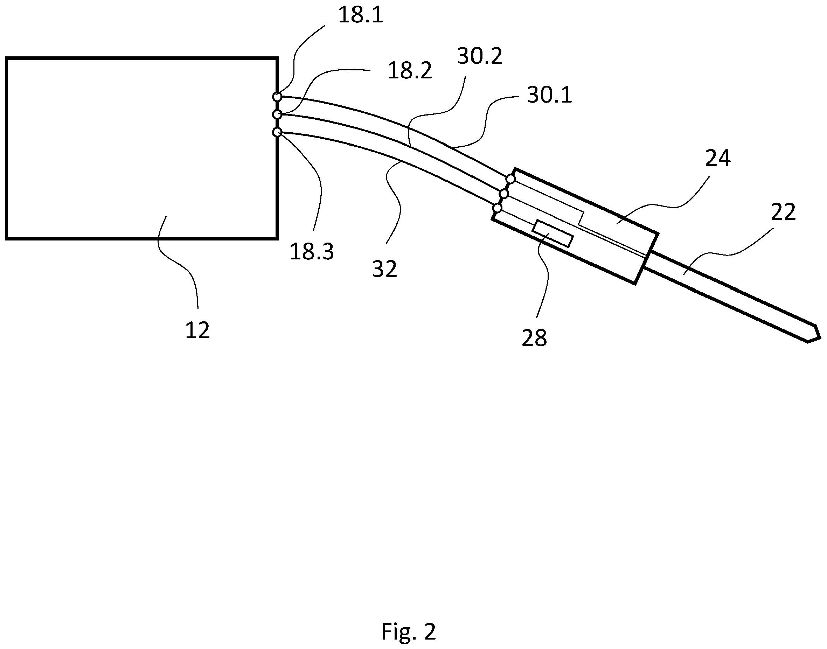

In dem Anschlusskabel 16 befinden sich sowohl Versorgungsleitungen 30.1 und 30.2 als auch wenigstens eine Datenleitung 32. Die Versorgungsleitungen 30.1 und 30.2 verbinden die aktive Elektrode 22 und eine nicht weiter dargestellte neutrale Elektrode mit den elektrischen Ausgängen 18.1 und 18.2 des Elektrochirurgie-Generators 12. Über die Datenleitung 32 ist der Datenspeicher 28 mit einem entsprechenden Anschluss 18.3 des Elektrochirurgie-Generators 12 verbunden. Dies ist schematisch in Figur 2 dargestellt.

Die Datenleitung 32 in dem Anschlusskabel 16 sowie auch der Anschluss 18.3 können mehradrig bzw. mehrpolig sein. Zusätzlich zu der Datenleitung 32 oder anstelle der Datenleitung 32 kann auch eine drahtlose Schnittstelle für die Datenübertragung von dem elektrochirurgischen Instrument 14 zu dem Elektrochirurgie-Generator 12 vorgesehen sein. Eine solche drahtlose Schnittstelle kann beispielsweise eine Bluetooth Schnittstelle, eine NFC Schnittstelle oder eine RFID Schnittstelle sein.

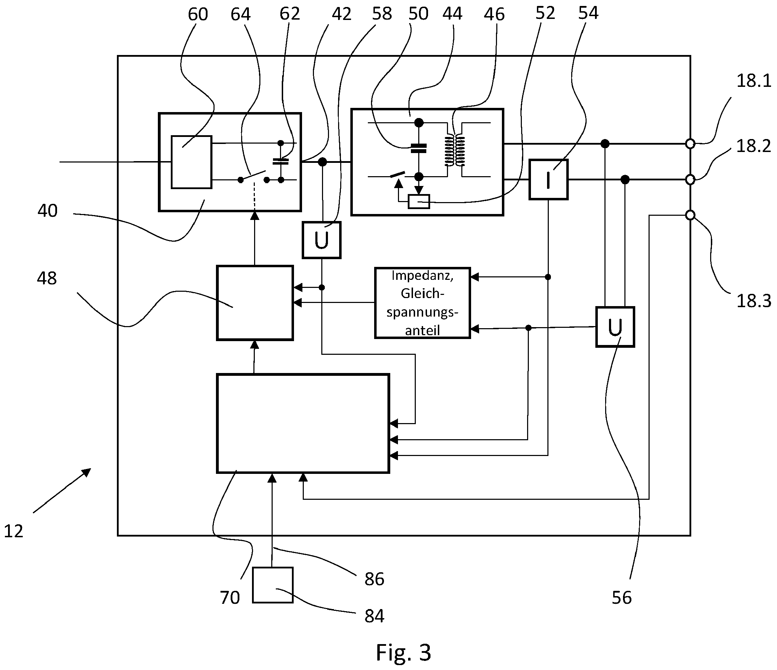

Die zum Betreiben des elektrochirurgischen Instruments 14 an dessen aktive Elektrode 22 und eine Gegenelektrode abzugebende Ausgangswechselspannung wird im Betrieb von dem Elektrochirurgie-Generator 12 bereitgestellt. Wie Figur 3 zu entnehmen ist, weist der Elektrochirurgie-Generator 12 hierzu ein Hochspannungsnetzteil 40 auf, das beispielsweise an das übliche öffentliche Stromversorgungsnetz angeschlossen werden kann und an seinem Ausgang 42 einen Hochspannungsgleichstrom mit einer Ausgangsgleichspannung bereitstellt. Dieser Ausgangsgleichstrom wird einem Hochfrequenzteil 44 des Elektrochirurgie-Generators 12 zugeführt. Der Hochfrequenzteil 44 des Elektrochirurgie-Generators 12 wirkt als Wechselrichter und erzeugt eine hochfrequente Ausgangswechselspannung, die über einen Ausgangstransformator 46 des Hochfrequenzteils 44 an die Ausgänge 18.1 und 18.2 des Elektrochirurgie-Generators 12 abgegeben wird. An die Ausgänge 18.1 und 18.2 des Elektrochirurgie-Generators 12 kann das elektrochirurgische Instrument 14 angeschlossen werden, wie es in den Figuren 1 und 2 dargestellt ist.

Um die Ausgangswechselspannung des Elektrochirurgie-Generators 12 zu steuern, ist eine Generatorsteuereinheit 48 vorgesehen, die die Ausgangswechselspannung an den Ausgängen 18.1 und 18.2 des Elektrochirurgie-Generators 12 auf Basis eines Ausgangswechselspannungsmaximalwertes derart steuert, dass beispielsweise ein eingestellter Ausgangsspannungsmaximalwert im Betrieb nicht überschritten wird.

Die Ausgangswechselspannung des Elektrochirurgie-Generators 12 - und damit auch der Ausgangswechselstrom und die Ausgangsleistung - können durch die von dem Hochspannungsnetzteil 40 erzeugte Ausgangsgleichspannung gesteuert werden.

Hierzu dient die Generatorsteuereinheit 48, die von dem Hochspannungsnetzteil 40 erzeugte Ausgangsgleichspannung so steuert, dass sich beispielsweise eine Ausgangswechselspannung oder ein Ausgangswechselstrom so ergibt, wie es ein jeweiliger Betriebsmodus des Elektrochirurgie-Generators zu einem jeweiligen Zeitpunkt verlangt.

Jeder Betriebsmodus definiert Maximalwerte für den Effektivwert der Ausgangswechselspannung über die Ausgänge 18.1 und 18.2, die Ausgangsspitzenspannung über die Ausgänge 18.1 und 18.2, den Effektivwert des Ausgangswechselstroms an den Ausgängen 18.1 oder 18.2, den Gleichspannungsanteil an der Ausgangswechselspannung über die Ausgänge 18.1 und 18.2 sowie die Ausgangsgleichspannung des Hochspannungsnetzteils 40. Die durch einen jeweiligen Betriebsmodus definierten Maximalwerte können dabei situationsabhängig sein und sich während einer Anwendung ändern.

Die Generatorsteuereinheit 48 steuert das Hochspannungsnetzteil 40 in Abhängigkeit von durch einen jeweiligen Betriebsmodus definierten Maximalwerten und von im Betrieb von Erfassungseinheiten 54, 56 und 58 erfassten aktuellen Werten der Ausgangswechselspannung, der Ausgangsspitzenspannung, des Ausgangswechselstroms, des Gleichspannungsanteil an der Ausgangswechselspannung oder der Ausgangsgleichspannung oder einer Kombination von Werten dieser Parameter.

Die konkreten Maximalwerte und der zeitliche Ablauf für das Erzeugen der Ausgangswechselspannung des Elektrochirurgie-Generators 12 und deren Abhängigkeit von erfassten Momentanwerten hängen vom jeweiligen Betriebsmodus (Mode) ab, in dem der Elektrochirurgie-Generator 12 gerade betrieben wird.

Ein Betriebsmodus wird beispielsweise durch Betätigen eines entsprechenden Schalters, beispielsweise einem Fußschalter 84 durch einen Nutzer aufgerufen.

In einem jeweiligen Betriebsmodus wird der Betrieb des Elektrochirurgie-Generators 12 von einem Prozessor 70 in Verbindung mit einem Betriebsprogramm 74 gesteuert, das in einem Generator-Datenspeicher 72 hinterlegt ist, mit dem der Prozessor 70 verbunden ist. Der Prozessor 70 bildet in Verbindung mit dem Betriebsprogramm 74 beispielsweise die Maximalwerte für die verschiedenen Betriebsparameter, wie die Ausgangswechselspannung, den Ausgangswechselstrom, die Ausgangsleistung oder aber auch den Gleichspannungsanteil an der Ausgangswechselspannung, deren jeweils aktuelle Werte während des Betriebs des Elektrochirurgie-Generators 12 erfasst werden.

Während des Ablaufs des in dem Generator-Datenspeicher 72 hinterlegten Betriebsprogramms 74 greift der Prozessor 70 an in dem Betriebsprogramm hinterlegten Stellen auf für einen jeweiligen Betriebsmodus ebenfalls in dem Generator-Datenspeicher 72 hinterlegte Betriebsvorgaben 76 wie beispielsweise Daten, die Werte für Betriebsparameter repräsentieren und/oder Steuerbefehle zurück. Die in dem Generator-Datenspeicher 72 hinterlegten Betriebsvorgaben 76 geben beispielsweise bestimmte Werte für die Ausgangsgleichspannung des Hochspannungsnetzteils oder die Ausgangswechselspannung, den Ausgangswechselstrom des Hochfrequenzteils oder Ähnliches vor. In dem Generator-Datenspeicher 72 hinterlegte Betriebsvorgaben 76 schließen aber auch bestimmte Steuerbefehle ein, wie beispielsweise *"if"* oder *"while"*oder *"true"* oder *"false".* Auf diese Weise können mit den in dem Generator-Datenspeicher 72 als Betriebsvorgaben hinterlegten Daten und Steuerbefehle beispielsweise solche Steueranweisungen definiert werden wie *"vergleiche den aktuellen Wert der Ausgangswechselspannung mit dem Betrag 200 und gib ein "true" aus, wenn der aktuelle Wert der Spannung kleiner oder gleich 200 ist und ein "false", wenn der aktuelle Wert größer ist als 200".* Andere Daten und Steuerbefehle können beispielsweise zu der Steueranweisung Kombiniert werden, die besagt, dass die maximale Ausgangsgleichspannung des Hochspannungsnetzteils 100 Volt sein soll.

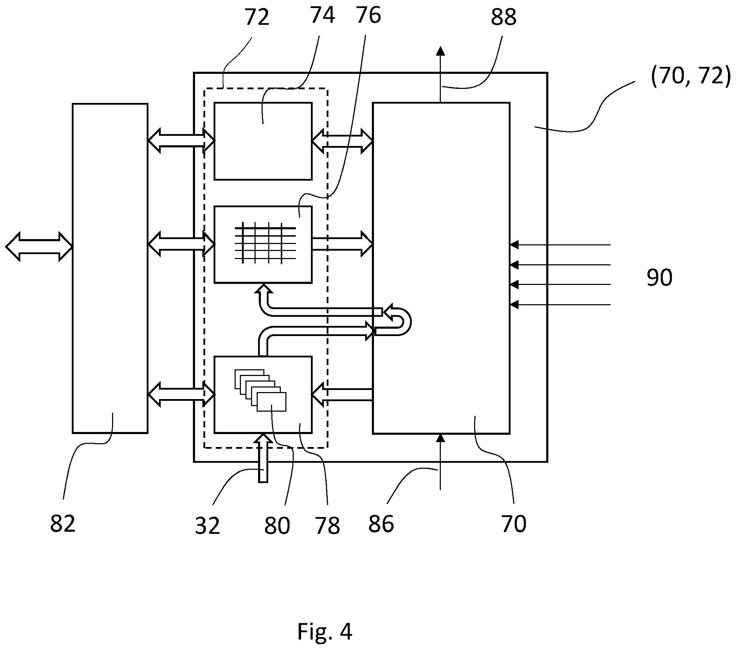

Um derartige Steueranweisungen zu generieren und auszuführen, greift der Prozessor 70 jedoch nicht direkt auf die Betriebsvorgaben 76 in dem Generator-Datenspeicher 72 zu, sondern ruft an entsprechenden Stellen des Betriebsprogramms 74 eine Datenstruktur 78 auf, in der für einen jeweiligen Betriebsmodus Verweise hinterlegt sind, die auf entsprechende Betriebsvorgaben 76 in dem Generator-Datenspeicher 72 verweisen, siehe z.B. Figur 4. Die Verweise sind 1-Byte groß, da sie somit wenig Speicherplatz erfordern. In diesem Falle können die Betriebsvorgaben 76 nach Art einer Tabelle strukturiert sein, in der jedem Verweis (also beispielsweise jeder Hexadezimalzahl) die entsprechenden Steuerbefehle oder Daten zugeordnet sind.

Um verschiedene Betriebsmodi zu verwirklichen, sind in der Datenstruktur 78 eine Vielzahl von Datensätzen 80 hinterlegt, von denen jeder ein oder mehrere Verweise enthält, denen aufgrund der Struktur des jeweiligen Datensatzes - insbesondere der Reihenfolge, in der die Verweise gespeichert sind - Zeilennummern zugeordnet werden können. Die einer Zeilennummer zugeordneten Verweise verweisen auf die entsprechenden Betriebsvorgaben 76 in dem Generator-Datenspeicher 72 und veranlassen den Prozessor 70, die entsprechenden Betriebsvorgaben 76 aus dem Generator-Datenspeicher 72 auszulesen, nachdem der Prozessor 70 zuvor auf die in der Datenstruktur 78 hinterlegte und die zugehörige Zeilennummer bezeichnete Einsprungadresse zugegriffen hat. Die in dem Generator-Datenspeicher 72 gespeicherten Betriebsvorgaben können beispielsweise Steueranweisungen, Steuerbefehle oder Parameterwerte sein, welche das Betriebsprogramm an der jeweiligen Stelle des Betriebsprogramms anwenden soll, an der sich in dem Betriebsprogramm 74 ein Verweis auf eine Zeilennummer in der Datenstruktur 78 befindet.

In der Datenstruktur 78 sind somit Verweise auf in dem Generator-Datenspeicher 72 gespeicherte Betriebsvorgaben 76 in einer geordneten Sequenz abgespeichert. Die Struktur eines jeweiligen Datensatzes in der Datenstruktur 78 erlaubt es, den Verweisen Zeilennummern nach Art von Adressen innerhalb der Datenstruktur 78 zuzuweisen, damit beispielsweise auch Sprünge oder Rücksprünge auf Verweise in der Datenstruktur 78 möglich sind und nicht lediglich ein streng sequentielles Abarbeiten der Verweise durch das Betriebsprogramm. Zeilennummern können als Einsprungadressen innerhalb der strukturierten Daten in der Datenstruktur 78 dienen, auf die der Prozessor 70 durch das Betriebsprogramm 74 gesteuert zugreift. Die den Zeilennummern zugeordneten Verweise verweisen auf entsprechende Betriebsvorgaben 76 in dem Generator-Datenspeicher 72. Diese Betriebsvorgaben 76 sind beispielsweise einzelne Steuerbefehle, zusammengesetzte Steueranweisungen oder Parameterwerte für Betriebsparameter. Die durch die parametrisierten Verweise in der Datenstruktur 78 aufgerufenen Betriebsvorgaben 76 steuern den Betrieb des Elektrochirurgie-Generators 12 in dem jeweiligen Betriebsmodus in Kombination mit dem Betriebsprogramm 74. Die Betriebsvorgaben können auch Steuerbefehle sein, die den Aufruf anderer Verweise in der Datenstruktur 78 bewirken. Allerdings ist dies nur innerhalb der Daten - also innerhalb derjenigen Verweise möglich, die zu einem jeweiligen Betriebsmodus gehören.

Die Verweise sind vorzugsweise durch Hexadezimalzahlen repräsentiert, die zusammen strukturierte Daten eines Datensatzes 80 bilden. Ein Datensatz gehört zu einem Betriebsmodus und kann zum Beispiel folgendes enthalten:

| | | |
|---|---|---|
| *006F* | **11** | |
| *0070* | **36** | 02 30 00 C8 00 |
| *0076* | **12** | |
| *0077* | **0F** | 04 64 00 |
| *0078* | **56** | |
| *007C* | **4B** | 03 02 |
| *007F* | **13** | |

Die kursiv dargestellten Zahlen sind beim Auslesen des Datensatzes 80 generierte Zeilennummern und sind nicht in dem Datenspeicher 28 des elektrochirurgischen Instrument 14 gespeichert, sondern werden von dem Betriebsprogramm entsprechend der Reihenfolge der Verweise in einem jeweiligen Datensatz selbst erzeugt. Die Zeilennummern ergeben sich somit durch die Reihenfolge der Verweise (also deren Struktur) in einem jeweiligen Datensatz. Die im Beispiel als kursiv dargestellte Zahlen dargestellten Zeilennummern sind einfach aufsteigende Zahlen entsprechend der Länge der strukturierten Daten. Die fett dargestellten Zahlen dienen als Verweise (oder Zeiger bzw. Pointer), die jeweils auf eine Betriebsvorgabe in dem Generator-Datenspeicher 72 verweisen, und zwar in dem dargestellten Beispiel auf Steuerbefehle. So verweist z.B. "11" auf den Steuerbefehl "IF", "36" auf einen Vergleich, der durch die nachfolgenden, zugeordneten Zahlen "02 30 00 C8 00" spezifiziert wird, "12" auf den Steuerbefehl "THEN", "0F" auf einen Steuerbefehl zum Setzen eines durch die nachfolgenden, zugeordneten Zahlen "04 64 00" spezifizierten Parameterwertes und "56" auf den Steuerbefehl "ELSE".

Aus der jeweiligen mittels parametrisiertem Verweis aufgerufenen Betriebsvorgabe 76 ergibt sich auch, welche Zusatzinformationen (wie z.B. "02 30 00 C8 00" in dem obigen Beispiel) noch relevant sind.

Das Betriebsprogramm 74 kann das vorstehend dargestellte Beispiel wie folgt lesen und übersetzen:
**11** - if -> keine Zusatzinfos erforderlich
**36** - Vergleich -> 5 Zeichen an Zusatzinformationen erforderlich (02 = 1 Zeichen Vergleichsart, 30 00 = 2 Zeichen Zahl 1, C8 00 = 2 Zeichen Zahl 2)
**12** - then -> keine Zusatzinfos erforderlich
**0F** - Ausgangswert setzen -> 3 Zeichen Zusatzinformationen (04 = 1 Zeichen Welcher Ausgangswert; 64 00 = 2 Zeichen Setzwert)
Übersetzt kann dies bedeuten " *Vergleiche, ob Zahl 1 (30 00) größer ist, als Zahl 2 (C8 00). Wenn das Ergebnis TRUE ist, dann setze Spannung auf 100 V (64 00), ansonsten* ..."

Die in dem Beispiel als Hexadezimalzahlen dargestellten Binärzahlen (und hier verkürzt als "Hexadezimalzahlen" bezeichneten Zahlen) in einem jeweiligen Datensatz 80 repräsentieren somit zum einen Verweise, auf deren Basis das Betriebsprogramm 74 auf Betriebsvorgaben 76 in dem Generator-Datenspeicher 72 zugreifen kann. Die in einem Datensatz strukturiert (geordnet) abgelegten Hexadezimalzahlen sind die vorgenannten Verweise auf in dem Generator-Datenspeicher 72 gespeicherte Betriebsvorgaben 76 wie z.B. Steuerbefehle und Parameter, denen aufgrund der Struktur des Datensatzes Zeilennummern zugeordnet werden können, die selbst nicht explizit im Datensatz 80 gespeichert sein müssen, sondern von dem Betriebsprogramm 74 beim Einlesen eines jeweiligen Datensatzes 80 erzeugt werden können.

Die in einem jeweiligen Datensatz 80 gespeicherten und in dem Beispiel als Hexadezimalzahlen dargestellten Binärzahlen dienen als Pointer (oder Zeiger), die jeweils auf eine konkrete Betriebsvorgabe 76 in dem Generator-Datenspeicher 72 des Elektrochirurgie-Generators 12 verweisen, so dass die entsprechende Hexadezimalzahl mit einer entsprechenden konkrete Betriebsvorgabe 76 in dem Generator-Datenspeicher 72 des Elektrochirurgie-Generators 12 verknüpft ist. Diese Hexadezimalzahlen werden somit zur Bezeichnung eines entsprechenden, eine Betriebsvorgabe 76 repräsentierenden Speichereintrags in dem Generator-Datenspeicher 72 des Elektrochirurgie-Generators 12 verwendet. Diese Hexadezimalzahlen sind somit eine Art Pointer, um das Betriebsprogramm 74 zu Speichereinträgen in dem Generator-Datenspeicher 72 des Elektrochirurgie-Generators 12 zu leiten, wo das Betriebsprogramm jeweils eine Betriebsvorgabe für das Betriebsprogramm während seiner Ausführung abrufen kann. Der Aufruf der Verweise erfolgt durch das Betriebsprogramm gesteuert und führt dazu, dass der Prozessor 70 die Speichereinträge in dem Generator-Datenspeicher 72 abruft, auf die die Verweise verweisen. Dabei sind auch Sprünge innerhalb der Verweise möglich, die durch ihre Sequenz oder Zeilennummernidentifiziert sind. Die strukturierten Daten in einem Datensatz können so über die ihnen entsprechenden Speichereinträge in dem Generator-Datenspeicher 72 in Betriebsvorgaben für das Betriebsprogramm übersetzt werden.

In dem vorstehend beispielhaft wiedergegebenen Datensatz stellen die Einträge in der ersten Spalte *("006F, 0070, 0076...")* Zeilennummern dar. Die Zeilennummern sind nicht in dem Instrumenten-Datenspeicher 28 des elektrochirurgischen Instrument 14 gespeichert, sondern werden von dem Betriebsprogramm 74 selbst erzeugt, da die Zeilennummern einfach aufsteigende Zahlen entsprechend der Länge der strukturierten Daten sind:
*0070* 36 02 30 00 C8 00 (Länge der Daten = 6, d.h. nächste Zeilennummer ist *0076)*
*0076* 12 (Länge der Daten = 1, d. h. nächste Zeilennummer ist *0077)*

Die Einträge in jeder Zeile ("**11**, **36** 02 30 00 C8 00, **12**...") verweisen auf Betriebsvorgaben 76 in dem Generator-Datenspeicher 72. Der durch 11 bezeichnete Eintrag in dem Generator-Datenspeicher 72 kann zum Beispiel eine "IF"-Anweisung sein, während der durch 12 bezeichnete Eintrag eine "THEN"-Anweisung sein kann. "IF"-Anweisung und "THEN"-Anweisung sind jeweils eine Betriebsvorgabe. Die in den strukturierten Daten des abgebildeten Datensatzes zwischen 11 und 12 stehenden Hexadezimalzahlen "**36** 02 30 00 C8 00" können aus den dazugehörigen Speichereinträgen in dem Generator-Datenspeicher 72 in eine Steueranweisung übersetzt werden, wie zum Beispiel *"Vergleiche den letzten abgelesenen Wert der Spannung (30 00) mit der Zahl 200 (C8 00), und wenn die Spannung kleiner oder gleich 200 ist, ist das Ergebnis "TRUE", andernfalls ist das Ergebnis "FALSE"'.* Wenn das Betriebsprogramm 74 an der (vom Betriebsprogramm erzeugten) Adresse *"0077'* die Speichereinträge für die Zeichenfolge **"0F** 04 64 00" aus dem Generator-Datenspeicher 72 abruft, könnte dies eine Einstellung für den Elektrochirurgie-Generator 12 bezeichnen; die Einstellung kann z.B. sein, dass ein Maximalwert für die Ausgangsgleichspannung (0F 04) auf 100V (64 00) eingestellt ist.

Die Steueranweisungen, die die Absaugeinrichtung 26 betreffen, können beispielsweise ein Ein- oder Ausschalten der Absaugeinrichtung oderfürden eingeschalteten Zustand der Absaugeinrichtung die jeweils durch die Absaugeinrichtung abzugebende Leistung definieren.

Der Elektrochirurgie-Generator 12 ist ausgebildet, solche Steueranweisungen, die die von der Absaugeinrichtung 26 abzugebende Leistung definieren, in Steuereingriffe in Form von Impulsfolgen umzusetzen, die im Sinne einer Pulsweitenmodulation ein schnell aufeinanderfolgendes Ein- und Ausschalten der Absaugeirichtung 26 bewirken, so dass diese im Ergebnis mit einer größeren oder kleineren Leistung betrieben wird. Eine im Rahmen der Pulsweitenmodulation abgegebene Folge schnell aufeinander folgender Einschalt-Impulse ist durch die Länge der Einschaltimpulse und die Länge der Pausen (Ausschaltzeiten) zwischen den einzelnen Einschaltimpulsen definiert. Je kürzer die Pausen (Ausschaltzeiten) und je länger die Einschaltimpulse sind, desto höher ist die im Ergebnis von der Absaugeinrichtung abgegebene Leistung.

Die in der Datenstruktur 78 gespeicherten strukturierten Daten 80 enthalten Einträge, die die Steueranweisungen definieren, die im Betrieb des Elektrochirurgie-Generators 12 in Steuereingriffe an der Absaugeinrichtung 26 umgesetzt werden.

In der abgebildeten bevorzugten Ausführungsvariante ist der Elektrochirurgie-Generator 12 dazu ausgebildet, die von der Absaugeinrichtung 26 im Betrieb abgegebene Leistung in Abhängigkeit eines berechneten oder eines sensorisch erfassten Parameterwertes zu regeln. Hierzu kann der Elektrochirurgie-Generator 12 über Leistungserfassungsmittel zum Erfassen der tatsächlich von dem Elektrochirurgie-Generator 12 abgegebenen Leistung aufweisen und die Absaugeinrichtung 26 entsprechend steuern.

Der Betrieb des Elektrochirurgie-Generators 12 und der, an ihn angeschlossen Absaugeinrichtung 26 in einem jeweiligen Betriebsmodus hängt somit zum einen von dem in dem Generator-Datenspeicher 72 gespeicherten Betriebsprogramm 74 ab. Darüber hinaus hängt das Betriebsverhalten des Elektrochirurgie-Generators 12 in einem jeweiligen Betriebsmodus aber auch von dem für einen jeweiligen Betriebsmodus aufgerufenen Datensatz 80 in der Datenstruktur 78 ab sowie von den im dem Generator-Datenspeicher 72 gespeicherten Betriebsvorgaben 76.

Der Vorteil eines solchen Elektrochirurgie-Generators 12 ist, dass neue Betriebsmodi und dazu passende Ansteuerungen der Absaugeinrichtung 26 leicht durch Erzeugen neuer Datensätze 80 in der Datenstruktur 78 definiert werden können und beispielsweise ein einzelner Betriebsmodus allein durch Ändern des entsprechenden Datensatzes 80 in der Datenstruktur 78 geändert werden kann, ohne dass das Betriebsprogramm 74 in dem Generator-Datenspeicher 72 oder die Betriebsvorgaben 76 in dem Generator-Datenspeicher 72 geändert werden müssen. Auf der anderen Seite können globale Parameter, wie beispielsweise die möglichen zur Verfügung stehenden Steueranweisungen oder von dem jeweiligen Elektrochirurgie-Generator abhängigen Betriebsparameter, wie dessen maximale Ausgangswechselspannung oder eine minimal zulässige Ausgangsgleichspannung als Betriebsvorgaben 76 in dem Generator-Datenspeicher 72 hinterlegt sein und dort auch gegebenenfalls zentral für alle möglichen Betriebsmodi zugleich geändert werden.

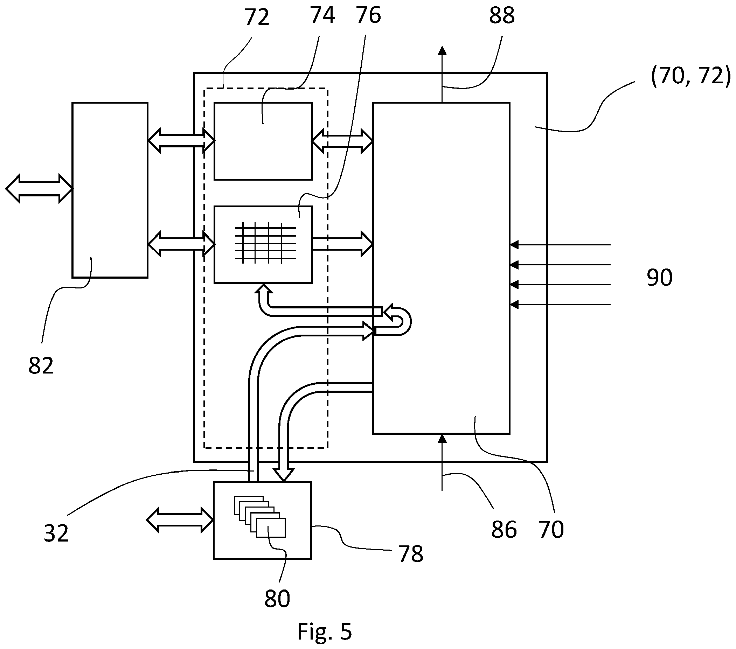

Ein weiterer Vorteil des Aufbaus des Elektrochirurgie-Generators 12 ist, dass ein Datensatz 80, dessen strukturierte Daten indirekt einen für das elektrochirurgische Instrument 14 geeigneten Betriebsmodus definieren, auch in einem elektrochirurgischen Instrument 14 hinterlegt sein kann; siehe Figur 5. Konkret kann der Instrumenten-Datenspeicher 28 des elektrochirurgischen Instruments 14 einen Datensatz 80 mit strukturierten Daten enthalten, die zu der Datenstruktur 78 kompatibel sind und genau wie andere strukturierte Daten in der Datenstruktur 78 durch entsprechende Verweise auf Betriebsvorgaben 76 in dem Generator-Datenspeicher 72 indirekt einen jeweiligen Betriebsmodus definieren.

Der Elektrochirurgie-Generator 12 ist daher so konfiguriert, dass er beim Anschließen eines elektrochirurgischen Instruments 14 jeweils - soweit vorhanden - zunächst dessen Instrumenten-Datenspeicher 28 ausliest und die strukturierten Daten aus dem in dem Datenspeicher 28 gespeicherten Datensatz 80 in die Datenstruktur 78 einträgt. Auf diese Weise steht dem Elektrochirurgie-Generator 12 dann im Betrieb ein genau auf das jeweilige elektrochirurgische Instrument 14 zugeschnittener Betriebsmodus zur Verfügung.

Um einen Zugriff auf die Inhalte des Instrumenten-Datenspeichers 28 des elektrochirurgischen Instruments 14 zu erlauben, ist wenigstens die Datenleitung 22 mit einem zugehörigen Anschluss 18.3 vorgesehen. Alternativ oder zusätzlich kann auch eine drahtlose Schnittstelle wie beispielsweise eine Bluetooth Schnittstelle oder eine NFC Schnittstelle für den Zugriff auf die Inhalte des Instrumenten-Datenspeichers 28 des elektrochirurgischen Instruments 14 vorgesehen sein.

Beim Übertragen der strukturierten Daten von dem Instrumenten-Datenspeicher 28 des elektrochirurgischen Instruments 14 in einem entsprechenden Datensatz in der Datenstruktur 78 des Elektrochirurgie-Generators 12 können ggf. die Zeilennummern passend zu dem Betriebsprogramm 74 des Elektrochirurgie-Generators 12 generiert werden.

Von großem Vorteil ist, dass der in dem Instrumenten-Datenspeicher 28 gespeicherte Datensatz nur strukturiert geordnete Verweise (Pointer auf weitere Speichereinträge in dem Generator-Datenspeicher 72) enthält, jedoch nicht unmittelbar irgendwelche Steueranweisungen oder Betriebsparameter für einen jeweiligen Betriebsmodus, da die Steueranweisungen und die Betriebsparameter als Betriebsvorgaben 76 in dem Generator-Datenspeicher 72 des Elektrochirurgie-Generators 12 zentral gespeichert sind.

Alternativ kann der Elektrochirurgie-Generator 12 auch so konfiguriert sein, dass er während des Betriebs ― also während des Ablaufs des Betriebsprogramms ― den Instrumenten-Datenspeicher 28 des elektrochirurgischen Instruments direkt 14 ausliest. Dies ist bei dem in Figur 5 dargestellten Beispiels der Fall. In diesem Fall ist es nicht nötig, dass die strukturierten Daten des Datensatzes in dem Instrumenten-Datenspeicher 28 des elektrochirurgischen Instruments 14 zunächst in die Datenstruktur 78 auf dem Elektrochirurgie-Generator 12 übertragen werden. Die zu generierenden Zeilennummern müssen dann allerdings zu den entsprechenden Aufrufen in dem Betriebsprogramm und den Einträgen in dem Generator-Datenspeicher 72 passen.

Ein Vorteil eines Elektrochirurgie-Systems 10 der hier beschriebenen Art ist, dass verschiedene, den Betrieb des Elektrochirurgie-Generators 12 bestimmende Parameterwerte und Betriebsvorgaben unabhängig voneinander gepflegt werden können. So ist das in dem Generator-Datenspeicher 72 gespeicherte Betriebsprogramm 74 unabhängig von den Betriebsvorgaben 76 in dem Generator-Datenspeicher 72 gespeichert. Betriebsvorgaben 76 sind wiederum unabhängig von den strukturierten Daten in der Datenstruktur 78 gespeichert.

Vorzugsweise ist eine Programmierschnittstelle 82 vorgesehen, die vorzugsweise eine graphische Benutzeroberfläche bereitstellt, über einen Datensatz mit strukturierten Daten, die als eine Vielzahl von parametrisierten Verweisen ausgeführt sind, erstellt werden und in dem Instrumenten-Datenspeicher 26 des elektrochirurgischen Instruments 14 abgelegt werden kann.

Vorzugsweise ist die Programmierschnittstelle 82 so konfiguriert, dass sie unterschiedlichen Nutzern unterschiedliche Rechte einräumt. So können die Rechte für das Programmieren des Betriebsprogramms 74, das in dem Generator-Datenspeicher 72 gespeichert ist, für das Eingeben der Betriebsvorgaben 76, die ebenfalls in dem Generator-Datenspeicher 72 gespeichert sind und für die strukturierten Daten, die in der Datenstruktur 78 gespeichert sind, unterschiedlich vergeben werden. Insbesondere kann auf diese Weise sichergestellt werden, dass Änderungen an den Betriebsvorgaben 76 oder Änderungen an dem Betriebsprogramm 74 nur von Entwicklern vorgenommen werden können, die mit dem jeweiligen Elektrochirurgie-Generator 12 vertraut sind. Die Programmierung des Betriebsprogramms 74 und der Betriebsvorgaben 76 kann somit durch Entwickler erfolgen, die mit dem jeweiligen Elektrochirurgie-Generator 12 vertraut sind, während die Definition von Betriebsmodi für ein elektrochirurgisches Instrument 14 durch Schaffen eines entsprechenden Datensatzes mit strukturierten Daten durch einen mit dem elektrochirurgischen Instrument 14 vertrauten Entwickler erfolgen kann. Vorzugsweise werden von einem mit dem elektrochirurgischen Instrument 14 vertrauten Entwickler erzeugte Datensätze in dem Instrumenten-Datenspeicher 28 des entsprechenden elektrochirurgischen Instruments 14 gespeichert, während weitere Betriebsmodi auch direkt in der Datenstruktur 78 auf dem Elektrochirurgie-Generator 12 gespeichert werden können. Hierzu kann der Elektrochirurgie-Generator 10 beispielsweise eine USB-Programmierschnittstelle aufweisen. Für in dem Instrumenten-Datenspeicher 28 des entsprechenden elektrochirurgischen Instruments 14 gespeicherte strukturierte Daten steht entweder die Datenleitung 32 in dem Anschlusskabel 16 mit entsprechender Schnittstelle zur Verfügung - oder, alternativ oder zusätzlich - auch eine drahtlose Schnittstelle wie beispielsweise eine Bluetooth Schnittstelle oder eine NFC Schnittstelle. Die strukturierten Daten aus dem Datensatz in dem Instrumenten-Datenspeicher 28 des elektrochirurgischen Instruments 14 können dann beim Anschließen des elektrochirurgischen Instruments 14 in die Datenstruktur 78 auf dem Elektrochirurgie-Generator 12 übertragen werden.

Dies ist insbesondere in Bezug auf verschiedene elektrochirurgische Instrumente 14 von Bedeutung, da die elektrochirurgischen Instrumente 14 möglicherweise von anderen Entwicklern integriert werden als der Elektrochirurgie-Generator 12. Die Entwickler des Elektrochirurgie-Generators 12 können alle für den Elektrochirurgie-Generator 12 wichtigen spezifischen Parameterwertedaten und Steuerbefehle - gegebenenfalls in Abhängigkeit von dem in dem Generator-Datenspeicher 72 gespeicherten Betriebsprogramm - als Betriebsvorgaben 76 in dem Generator-Datenspeicher 72 ablegen. Solche Parameterwerte können beispielsweise maximal oder minimal zulässige Werte für die Ausgangsgleichspannung, die Ausgangswechselspannung, etc. sein.

Davon unabhängig, können Entwickler eines elektrochirurgischen Instruments 14 mithilfe der strukturierten Daten in dem Datensatz in dem Instrumenten-Datenspeicher 28 des elektrochirurgischen Geräts genau vorgeben, wie ein spezifischer Betriebsmodus für dieses elektrochirurgische Instrument 14 im Rahmen der durch das Betriebsprogramm 74 in dem Generator-Datenspeicher 72 und die Betriebsvorgaben 76 in dem Generator-Datenspeicher 72 definierten Grenzwerte ablaufen kann. Dabei müssen sich die Entwickler des elektrochirurgischen Instruments 14 keine weiteren Gedanken über die Vorgaben in durch das Betriebsprogramm 74 und die Betriebsvorgaben 76 machen. Vielmehr können die Entwickler des elektrochirurgischen Instruments 14 diese für den jeweiligen Elektrochirurgie-Generator 12 gegebenen Vorgaben hinnehmen.

Den Entwicklern eines elektrochirurgischen Instruments 14 steht zum Definieren eines Betriebsmodus für das jeweilige elektrochirurgische Instrument 14 und die zugehörigen Steueranweisungen für den Betrieb der Absaugeinrichtung eine Programmierschnittstelle 82 zur Verfügung, über die ein Entwickler einen Betriebsmodus für ein jeweiliges elektrochirurgisches Instrument 14 vollständig definieren kann. Dies schließt beispielsweise alle Strom- und Spannungsparameterwerte ein sowie auch Zeitvorgaben und Übergangsbedingungen für den Betrieb des elektrochirurgischen Instruments. Auf diese Weise kann die Entwicklung des Betriebsmodus mithilfe eines einfach zu bedienenden Werkzeugs nahezu ohne Software-Entwicklungskenntnisse durch einen Entwickler für ein elektrochirurgisches Instrument erfolgen. Die Programmierschnittstelle 82 stellt dem Entwickler eine Reihe von Parametersätzen zur Verfügung, mit denen dieser verschiedene Phasen, zum Beispiel die Anschnittsphase, die Schneidphase, die Koagulationsphase, aber auch Kurzschluss- oder Leistungsüberwachungen für das jeweilige elektrochirurgische Instrument und die dazu passende Absaugleistung er Absaugeinrichtung 26 definieren kann. Ein zu der Programmierschnittstelle 82 gehörendes Entwicklungswerkzeug erzeugt aus den Vorgaben einen speicherplatzsparenden Satz strukturierter Daten, der einen Datensatz bildet, der in dem Datenspeicher 28 des elektrochirurgischen Instruments 14 nichtflüchtig hinterlegt werden kann.

Falls ein durch strukturierte Daten in einem Datensatz definierter, neuer Betriebsmodus für ein Elektrochirurgieinstrument oder eine veränderte Ansteuerung der Absaugeinrichtung auch eine Veränderung des Betriebsprogramms in dem Generator-Datenspeicher oder der Betriebsvorgaben in dem Generator-Datenspeicher 72 erfordert, können derartige Veränderungen beispielsweise von einem mit dem Elektrochirurgie-Generator vertrauten Entwickler über eine Programmierschnittstelle vorgenommen werden. So kann sichergestellt werden, dass die einen Betriebsmodus definierten strukturierten Daten zu den Betriebsvorgaben 76 in dem Generator-Datenspeicher 72 und zu dem Betriebsprogramm 74 in dem Generator-Datenspeicher 72 kompatibel sind.

Wird ein elektrochirurgisches Instrument 14 an den Elektrochirurgie-Generator 12 angeschlossen, liest der Elektrochirurgie-Generator 12 den Instrumenten-Datenspeicher 28 in dem elektrochirurgischen Instrument 14 aus und ruft die durch die in den strukturieren Daten enthaltenen Verweise bezeichneten Betriebsvorgaben 76 in dem Generator-Datenspeicher 72 auf, so dass die dort definierten Strom- und Spannungsverläufe inklusiver aller Zeitbedingungen und sonstiger Bedingungen - auch in Bezug auf die Absaugeinrichtung - angewandt werden. Auf diese Weise sind reduzierte Entwicklungszeiten und -kosten möglich. Das elektrochirurgische Instrument 14 kann weitestgehend unabhängig von einem Elektrochirurgie-Generator 12 integriert werden. Auch sind kürzere Produkteinführungszeiten möglich, da Betriebsmodi auch nach Einführen eines Elektrochirurgie-Generators 12 entwickelt und fertiggestellt werden können. Optimierungen eines Betriebsmodus und neuer Betriebsmodi können leicht durch aktualisierte oder neue elektrochirurgische Instrumente 14 eingeführt werden. Die Definition eines Betriebsmodus für ein elektrochirurgisches Instrument kann nahezu ohne Software-Entwicklungskenntnisse erfolgen.

Wenn das Elektrochirurgiesystem 10 mit einem an den Elektrochirurgie-Generator 12 angeschlossenen elektrochirurgischen Instrument 14 betrieben werden soll, steht der geeignete Betriebsmodus bereits nach Anschließen des elektrochirurgischen Instruments 14 zur Verfügung, weil die zugehörigen Datensätze 80 mit den strukturierten Daten von dem Instrumenten-Datenspeicher28 des elektrochirurgischen Instruments 14 ausgelesen werden können. Ein Nutzer muss daher nur noch beispielsweise einen Schalter 84 betätigen um das elektrochirurgische Instrument 14 in dem geeigneten Betriebsmodus des Elektrochirurgie-Generators 12 zu betreiben. Einstellungen oder Programmierungen muss der Nutzer nach Anschließen des elektrochirurgischen Instruments 14 nicht mehr vornehmen.

Der Schalter 84 ist über eine Leitung 86 mit dem Prozessor 70 des Elektrochirurgie-Generators 12 verbunden, so dass mit Betätigen des Schalters 84 der Ablauf des in dem Generator-Datenspeicher 72 gespeicherten Betriebsprogramms 74 gestartet und unterbrochen werden kann. Der Schalter 84 kann ein Fußschalter sein, er kann aber auch ein Handschalter sein, der sich beispielsweise an dem elektrochirurgischen Handinstrument 14 befindet. Anstelle der Leitung 86 kann auch hier eine drahtlose Steuerverbindung vorgesehen sein.

Eine weitere Alternative zu einem Schalter 84 ist ein automatisches Starten des Betriebsprogramms, das ein Benutzer vorab aktivieren kann. In diesem Fall wird von dem elektrochirurgischen Instrument zunächst eine kleine Messspannung ausgegeben, um mit deren Hilfe einen Gewebekontakt (Stromfluss) zu detektieren. Wird ein Gewebekontakt - also ein Stromfluss - detektiert, wird das eigentliche Betriebsprogramm für das elektrochirurgische Instrument aufgerufen. Verschwindet der Gewebekontakt, wird das eigentliche Betriebsprogramm für das elektrochirurgische Instrument beendet, und es wird wieder eine kleine Messspannung ausgegeben, um mit deren Hilfe einen erneuten Gewebekontakt zu detektieren.

Durch das Betriebsprogram gesteuert greift der Prozessor während der Anwendung eines Betriebsmodus indirekt auf einzelne Betriebsvorgaben 76 in dem Generator-Datenspeicher 72 zu, indem zunächst ein Zugriff auf Verweise in der Datenstruktur 78 erfolgt und daraufhin ein Abruf derjenigen Betriebsvorgabe oder Betriebsvorgaben erfolgt, auf die ein jeweiliger Verweis verweist.

Abhängig sowohl von dem Betriebsprogramm 74 als auch den Betriebsvorgaben 76 in dem Generator-Datenspeicher 72 als auch der Datenstruktur 78 sowie von Signalen 90, die von den Erfassungseinheiten 54, 56 und 58 stammen, generiert der Prozessor 70 Steuersignale 88 für die Generatorsteuereinheit 46.

### Bezugszeichen

- 10: Elektrochirurgiesystem
- 12: Elektrochirurgie-Generator
- 14: elektrochirurgisches Instrument
- 16: Anschlusskabel
- 18.1, 18.2: elektrischen Ausgänge
- 18.3: Anschluss
- 20: Schaft
- 20.1,20.2: Ausgänge
- 22: aktive Elektrode
- 24: Handgriff
- 26: Absaugeinrichtung
- 27: Steuerleitung
- 28: Datenspeicher
- 30.1,30.2: Versorgungsleitungen
- 32: Datenleitung
- 40: Hochspannungsnetzteil
- 42: Ausgang
- 44: Hochfrequenzteil
- 46: Ausgangstransformator
- 48: Generatorsteuereinheit
- 50: Kondensator
- 52: Synchronisierschaltung
- 54: Ausgangsstromerfassungseinheit
- 56: Ausgangswechselspannungserfassungseinheit
- 58: Ausgangsgleichspannungserfassungseinheit
- 60: Hochspannungsgleichrichterschaltung
- 62: Ausgangskondensator
- 64: Schalter
- 70: Prozessor
- 72: Generator-Datenspeicher
- 74: Betreibsprogramm
- 76: Betriebsvorgaben
- 78: Datenstruktur
- 80: Datensatz
- 84: Schalter
- 86: Leitung
- 88: Steuersignale des Prozessors
- 90: Signale der Erfassungseinheiten

## Patentansprüche

1. Elektrochirurgiesystem (10) mit einem elektrochirurgischen Instrument (14), einer Absaugeinrichtung (26) und einem Elektrochirurgie-Generator (12), an den das elektrochirurgische Instrument (14) und die Absaugeinrichtung (26) angeschlossen sind, wobei der Elektrochirurgie-Generator (12) einen Prozessor (70) und mindestens einem Generator-Datenspeicher (7e) aufweist und dazu konfiguriert ist, Einträge (74, 76, 78) in dem Generator-Datenspeicher (72) in Steueranweisungen für den Betrieb der Absaugeinrichtung (26) umzusetzen und im Betrieb den Steueranweisungen entsprechende Steuereingriffe zur Ansteuerung der Absaugeinrichtung (26) vorzunehmen, wobei die Steueranweisungen für den Betrieb der Absaugeinrichtung (26) definierenden Einträge in dem Speicher (74, 76, 78) für ein jeweiliges elektrochirurgische Instrument (14) und/oder einen jeweiligen Betriebsmodus spezifisch sind.

2. Elektrochirurgiesystem (10) gemäß Anspruch 1, bei dem der Generator-Datenspeicher (72) ein Betriebsprogramm (74), Betriebsvorgaben (76) und eine Datenstruktur (78) enthält, von denen
- das Betriebsprogramm (74) den Prozessor (70) zur Steuerung des Betriebs des Elektrochirurgie-Generators (12) in Verbindung mit dem elektrochirurgischen Instrument (14) veranlasst,
- die Betriebsvorgaben (76) im Betrieb des Elektrochirurgie-Generators (12) von dem durch das Betriebsprogramm gesteuerten Prozessor aufgerufen werden können und den Betrieb des Elektrochirurgie-Generator (12) beeinflussen können, aber keine festen Betriebsabläufe vorgeben und
- in der Datenstruktur (78) Datensätze (80) mit strukturierten Daten gespeichert sind, die Verweise auf in dem Generator-Datenspeicher (72) gespeicherte Betriebsvorgaben enthalten, die im Betrieb ein gezieltes Aufrufen einzelner Verweise durch Betriebsprogramm erlauben
wobei die strukturierten Daten Einträge enthalten, die Steueranweisungen für den Betrieb der Absaugeinrichtung (26) definieren.

3. Elektrochirurgiesystem (10) gemäß Anspruch 1 oder 2, bei dem das elektrochirurgische Instrument (14) einen nichtflüchtigen Instrumenten-Datenspeicher (28) enthält, der einen Datensatz mit strukturierten Daten enthält, die zu der Datenstruktur des Elektrochirurgie-Generators (12) kompatibel sind und die Einträge enthalten, die Steueranweisungen zum Ansteuern der Absaugeinrichtung (26) definieren.

4. Elektrochirurgiesystem (10) gemäß wenigstens einem der Ansprüche 1 bis 3, bei dem der Prozessor (70) mittels des Betriebsprogramms dazu konfiguriert ist, für unterschiedliche elektrochirurgische Instrumente (14) und/oder unterschiedliche Betriebsmodi jeweils spezifische Steueranweisungen einzulesen und deren Umsetzung in Steuereingriffe an dem Elektrochirurgie-Generator (12), an dem elektrochirurgischen Instrument (14) und/oder an der Absaugeinrichtung (26) zu bewirken.

5. Elektrochirurgiesystem (10) gemäß wenigstens einem der Ansprüche 1 bis 4, bei dem die durch gespeicherte Einträge in einem Speicher (74, 76, 78) definierten Steueranweisungen für Steuereingriffe an der Absaugeinrichtung (26) bedingte Steueranweisungen sind, die von und jeweils aktuellen Betriebszuständen des Elektrochirurgie-Generators und/oder von aktuell auftretenden Betriebsparameterwerten dynamisch abhängige Steuereingriffe definieren.

6. Elektrochirurgiesystem (10) gemäß wenigstens einem der Ansprüche 1 bis 5, bei dem die Steueranweisungen, die die Ansaugeinrichtung (26) betreffen, ein Ein- oder Ausschalten der Absaugeinrichtung oder für den eingeschalteten Zustand der Absaugeinrichtung die jeweils durch die Absaugeinrichtung abzugebende Leistung definieren.

7. Elektrochirurgiesystem (10) gemäß Anspruch 6, bei dem der Elektrochirurgie-Generator (12) dazu ausgebildet ist, solche Steueranweisungen, die die von der Absaugeinrichtung (26) abzugebende Leistung definieren, in Steuereingriffe in Form von Impulsfolgen umzusetzen, die im Sinne einer Pulsweitenmodulation ein schnell aufeinanderfolgendes Ein- und Ausschalten der Absaugeirichtung bewirken, so dass die Absaugeinrichtung (26) im Ergebnis mit einer größeren oder kleineren Leistung betrieben wird.

8. Elektrochirurgiesystem (10) gemäß wenigstens einem der Ansprüche 1 bis 7, bei dem der Elektrochirurgie-Generator (12) dazu ausgebildet ist, die von der Absaugeinrichtung (26) im Betrieb abgegebene Leistung in Abhängigkeit eines berechneten oder eines sensorisch erfassten Parameterwertes zu steuern.

9. Elektrochirurgiesystem (10) gemäß wenigstens einem der Ansprüche 1 bis 8, bei dem der Elektrochirurgie-Generator über Leistungserfassungsmittel zum Erfassen einer von einem elektrochirurgischen Instrument (14) abgegebenen Leistung aufweist.

10. Elektrochirurgiesystem (10) gemäß wenigstens einem der Ansprüche 1 bis 9, mit einer Programmierschnittstelle (82) oder mehreren Programmierschnittstellen, mittels der die Betriebsvorgaben (76) und die Datenstruktur (78) programmiert werden können.

11. Verfahren zum Betreiben eines Elektrochirurgiesystems (10) gemäß einem der Ansprüche 1 bis 10, **dadurch gekennzeichnet, dass** Einträge(74, 76, 78) in dem Generator-Datenspeicher (72) in Steueranweisungen für den Betrieb der Absaugeinrichtung (26) umgesetzt werden und im Betrieb den Steueranweisungen entsprechende Steuereingriffe zur Ansteuerung der Absaugeinrichtung (26) vorgenommen werden, wobei die Steueranweisungen für den Betrieb der Absaugeinrichtung (26) definierenden Einträge (74, 76, 78)in dem Generator-Datenspeicher (72) für ein jeweiliges elektrochirurgische Instrument (14) und/oder einen jeweiligen Betriebsmodus spezifisch sind.

12. Verfahren nach Anspruch 11, bei dem die die Steueranweisungen für den Betrieb der Absaugeinrichtung (26) definierenden Einträge aus einer Datenstruktur (78) aus einem Instrumenten-Datenspeicher (28) eines elektrochirurgischen Instruments (14) ausgelesen werden.

13. Elektrochirurgie-Generator (12) für ein Elektrochirurgiesystem (10) gemäß der Ansprüche 1 bis 10, mit einem Prozessor (70) und mindestens einem Generator-Datenspeicher (72), wobei der Prozessor (70) dazu konfiguriert ist, Einträge (74, 76, 78) in dem Generator-Datenspeicher (72) in Steueranweisungen für den Betrieb einer Absaugeinrichtung (26) umzusetzen und im Betrieb den Steueranweisungen entsprechende Steuereingriffe zur Ansteuerung der Absaugeinrichtung (26) vorzunehmen, wobei die Steueranweisungen für den Betrieb der Absaugeinrichtung (26) definierenden Einträge in dem Speicher (74, 76, 78) für ein jeweiliges elektrochirurgische Instrument (14) und/oder einen jeweiligen Betriebsmodus spezifisch sind.

14. Elektrochirurgie-Generator (12) gemäß Anspruch 13, **dadurch gekennzeichnet, dass** der Elektrochirurgie-Generator (12) dazu ausgebildet ist, solche Steueranweisungen, die die von der Absaugeinrichtung (26) abzugebende Leistung definieren, in Steuereingriffe in Form von Impulsfolgen umzusetzen, die im Sinne einer Pulsweitenmodulation ein schnell aufeinanderfolgendes Ein- und Ausschalten der Absaugeirichtung bewirken, so dass die Absaugeinrichtung (26) im Ergebnis mit einer größeren oder kleineren Leistung betrieben wird.

15. Elektrochirurgisches Instrument (14) für ein Elektrochirurgiesystem (10) gemäß der Ansprüche 1 bis 10, mit einem Instrumenten-Datenspeicher (28), der Datensätze (80) mit strukturierten Daten enthält, die Einträge enthalten, die Steueranweisungen für den Betrieb einer Absaugeinrichtung (26) definieren.
